# EUROPÄISCHE PATENTSCHRIFT

(11) **EP 2 510 972 B1**
(45) Veröffentlichungstag und Bekanntmachung des Hinweises auf die Patenterteilung: **06.08.2014**
(21) Anmeldenummer: 12159235.6
(22) Anmeldetag: 13.03.2012
(51) Int. Cl.: A61M 25/01, A61M 25/10

(54) **Kathetervorrichtung**
Catheter device
Dispositif de cathéter

(30) Priorität: 14.04.2011 US 201161475243 P
(43) Veröffentlichungstag der Anmeldung: 17.10.2012
(73) Patentinhaber: Biotronik AG, 8180 Bülach (CH)
(72) Erfinder: Wesselmann, Matthias, 8455 Rüdlingen (CH)
(74) Vertreter: Lindner-Vogt, Karin L.

(56) Entgegenhaltungen:
- WO-A1-2005/107643
- US-A- 6 039 721
- US-A1- 2009 018 501
- US-A1- 2009 204 083
- US-A1- 2011 054 438

## Beschreibung

Die Erfindung betrifft eine Kathetervorrichtung zur Aufweitung von Stenosen in Körpergefäßen.

Derartige Katheter sind in großer Vielgestaltigkeit seit Jahrzehenten bekannt und im klinischen Einsatz und haben sich in mannigfachen Anwendungssituationen bewährt. Eine jüngere Entwicklungsstufe stellen im Folgenden als "Hybridkatheter" bezeichnete Varianten dar, wie sie etwa aus der US 5,662,703 bekannt sind und bei denen der eigentliche Ballonkatheter noch um eine sog. Rollmembran ergänzt ist. Die Rollmembran dient in der erwähnten Druckschrift als Halteeinrichtung für einen röhrenförmigen, radial expandierbaren Stent, und dieser wird durch Betätigung der Rollmembran - insbesondere in etwas aufgeweitetem Zustand - an den Einsatzort gebracht und dort frei gegeben. Auch die EP 0820259 B1 offenbart einen derartigen Hybridkatheter, und auch dessen Konstruktion ist auf die Funktion des Verbringens eines Stents an seinen Einsatzort (hier im radial zusammengedrückten) Zustand angepasst. Obgleich die letztgenannte Kathetervorrichtung primär für den Einsatz mit selbstexpandierenden Stents vorgesehen ist, weist sie in einer Ausführungsform auch einen Dilatationskatheter auf.

WO2005/107643 offenbart einen Katheter zur Behandlung einer Bifurkation. Der Katheter umfasst ein proximales Ende, ein distales Ende und ein zweites distales Ende, wobei jedes distale Ende eine Spitze und einen Ballon aufweist. US2009/0018501 offenbart einen Katheter mit einer flexiblen Membran im distalen Bereich des Katheters. US2011/00584438 offenbart ein System und ein Verfahren zur Applikation eines Stents oder Scaffolds in einer Bifurkation eines Körpergefäßes. US6039721 offenbart ein Verfahren zur Behandlung mehrerer Stenosen verschiedener Länge mit einer Katheterprozedur. US2009/0204083 offenbart ein Verfahren und eine Vorrichtung zur Behandlung von Stenose im Bereich einer Bifurkation.

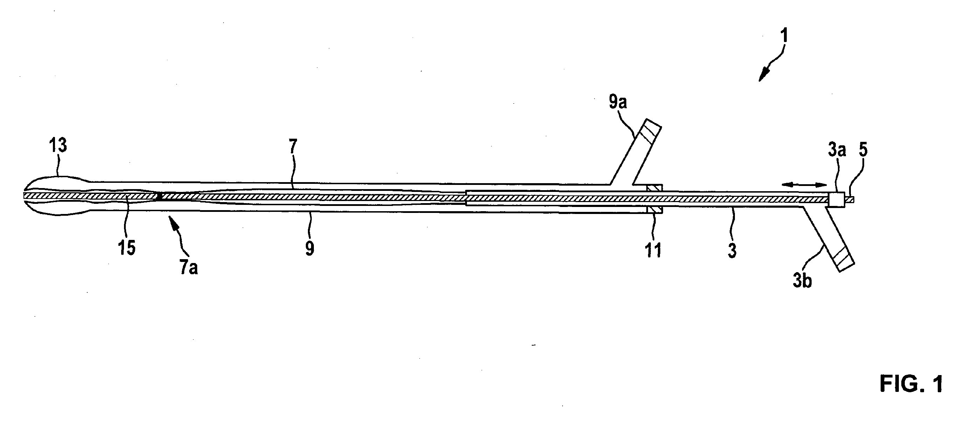

Fig. 1 zeigt einen Hybridkatheter 1 der hier in Rede stehenden Art in Form einer schematischen Längsschnittdarstellung. Der Katheter 1 hat einen Innenschaft 3 mit einem ersten proximalen Anschluss 3a zum Einführen eines Führungsdrahtes 5 und einem zweiten proximalen Luer-Anschluss 3b zur Beaufschlagung mit Druck. Am distalen Ende des Innenschaftes 3 ist ein aufweitbarer Ballon (Innenballon) 7 angebracht, der durch am Anschluss 3b anliegenden Druck aufgeweitet werden kann. Der Innenschaft 3 ist über den größten Teil seiner Länge von einem Außenschaft 9 umgeben, welcher an seinem proximalen Ende einen Dichteinsatz 11 mit niedriger Reibung zur abdichtenden Führung auf dem Außenumfang des Innenschafts 3 hat. Am distalen Ende des Außenschafts 9 ist eine Rollmembran 13 angebracht, welche außerdem, unter druckdichter Abtrennung von jenem, an einer Verbindungsstelle 13a mit dem distalen Ende des Innenballons fest verbunden ist. In Funktion gesetzt wird die Rollmembran 13, ähnlich wie der Innenballon 7, durch Druck, welcher an einem proximalen Lueranschluss 9a des Außenschafts 9 anliegt. Zu erwähnen ist, dass der Innenschaft 3 und Außenschaft 9 ein gemeinsames Führungsdrahtlumen 15 für den Führungsdraht 5 aufweisen.

Auch eine jüngere unveröffentlichte US-Patentanmeldung der Anmelderin beschreibt einen neuartigen Hybridkatheter, der sich durch einen speziellen Aufbau mit einem äußeren, einem inneren und einem mittleren Schaft und einer speziellen axialen Lagebeziehung zwischen dem Ballon und der Rollmembran auszeichnet. In der letztgenannten Druckschrift wird der Einsatz eines derartigen Katheters zum Aufweiten von Stenosen (unabhängig von Einsetzen eines Stents) beschrieben.

Der Erfindung liegt die Aufgabe zugrunde, eine verbesserte Kathetervorrichtung anzugeben, die insbesondere Vorteile bei der Aufweitung von Stenosen in Verzweigungsbereichen von Körpergefäßen erbringt und leicht und zuverlässig handhabbar ist.

Diese Aufgabe wird durch eine Kathetervorrichtung mit den Merkmalen des Anspruchs 1 gelöst. Zweckmäßige Fortbildungen des erfindungsgemäßen Gedankens sind Gegenstand der abhängigen Ansprüche. Beispielsweise wird des Weiteren ein Betriebsverfahren der Kathetervorrichtung vorgeschlagen.

Gemäß einem wesentlichen Gedanken der Erfindung umfasst die Kathetervorrichtung zwei Katheterzweige, die jeweils eine außenliegende und im Wesentlichen distal angeordnete Rollmembran aufweisen, in fester Zuordnung zueinander (vor und nach dem Durchqueren und Dilatieren der Stenose). Die feste Zuordnung der beiden Katheterzweige im Ruhezustand wird gemäß einem weiteren Aspekt der Erfindung durch einen gemeinsamen Katheterschaft oder fest verbundene Katheterschäfte verwirklicht, in dem beide Katheterzweige untergebracht sind. Ein weiterer Aspekt der Erfindung besteht darin, dass die beiden Katheterzweige an oder nahe dem distalen Ende eine geeignete Verbindung miteinander haben, welche auch bei Funktionsentfaltung erhalten bleibt und die beiden Zweige in einer vorbestimmten Zuordnung zueinander hält. Beide Katheterzweige weisen einen innenliegenden Ballonkatheterteil auf.

In einer weiteren Ausführung der Erfindung haben die beiden Katheterzweige unterschiedliche Funktionsabmessungen, insbesondere einen unterschiedlichen Nutzdurchmesser der jeweiligen Rollmembran und/oder eines Innenballons des jeweiligen Ballonkatheterteils. Eine zweckmäßige Ausgestaltung der letzteren Ausführung zeichnet sich durch verwechslungssichere Ausbildung der beiden Katheterzweige aus, die insbesondere durch Führungsdrahtlumen mit unterschiedlichem Durchmesser verwirklicht ist. Diese Ausgestaltung macht die Handhabung der vorgeschlagenen Kathetervorrichtung leichter und sicherer.

Die beiden Katheterzweige sind in einem Abschnitt des Umfangs am distalen Ende ihrer Außenschäfte 7a miteinander verbunden. Die Verbindung kann durch Verschweißen, Verkleben der Außenschäfte 7a oder auch durch einen gemeinsam benutzten Außenschaft 7a erfolgen. Dies hat eine gewisse Stabilisierung der Gesamtvorrichtung zur Folge und wirkt im Übrigen im Sinne der weiter oben angesprochenen Aufrechterhaltung einer vorbestimmten räumlichen Zuordnung zwischen den Katheterzweigen auch unmittelbar vor bzw. während der Ausführung ihrer Funktion.

In einer weiteren Ausführung der Erfindung sind Mittel zur axialen Lagefixierung des inneren Ballons 7 des oder jedes Ballonkatheterteils bezüglich der Längsachse der Kathetervorrichtung vorgesehen. Dies sind insbesondere ein Hinterschnitt im jeweiligen distalen Außenschaft eines Katheterastes, der es erlaubt, dass sich der Innenballon im Außenschaft beim Dilatieren leicht entfaltet und sich so "verkeilt". Alternativ oder zusätzlich kann noch der Innenschaft am proximalen Ende mittels einer Quetschdichtung (z.B. vom Typ Tuhoy Borst) axial arretiert werden. Diese Funktion ist besonders wichtig, da sie die flexible Anpassung der Ballonlängen ermöglicht.

In einer Ausführung der Erfindung sind die zwei Katheterzweige in ihrer Länge unabhängig voneinander einstellbar. Ebenfalls ist die Position der zwei Katheterzweige in der Arterie unabhängig voneinander einstellbar. Diese beiden Einstellmöglichkeiten machen es möglich, dass nur die Stenose in den Verzweigungsbereichen behandelt wird.

Gemäß einer weiteren Ausführung der Erfindung ist am Umfang der beiden Katheterzweige auf deren Rollmembranabschnitten eine Wirkstoffbeschichtung vorgesehen. Beim Ausrollen der Rollmembran wird zunächst der Blutstrom gestoppt, bevor der Wirkstoff freigesetzt wird. Dies verringert den Wirkstoffverlust, wodurch der Wirkstoff geringer dosiert werden kann. Eine Wirkstoffbeschichtung vermag insbesondere wegen der relativ hohen Andruckkräfte der Rollmembran an stenotische Gefäßabschnitte eine intensive Wirkstoffübertragung in die Stenose und somit einen starken therapeutischen Effekt eines geeigneten Wirkstoffes zu bewirken.

Vorteile und Zweckmäßigkeiten der Erfindung ergeben sich im Übrigen aus der Beschreibung eines Ausführungsbeispiels anhand der Figuren. Von diesen zeigen:
- Fig. 1: eine schematische Ansicht (vereinfachte Längsschnittdarstellung) eines Hybridkatheters,
- Fig. 2A bis 2D: das distale Ende einer Ausführungsform der Erfindung in verschiedenen Betriebszuständen an einer Gefäßverzweigung mit stenotischen Bereichen und
- Fig. 3: eine modifizierte Ausführung der Ballonkonstruktion einer erfmdungsgemäßen Kathetervorrichtung.

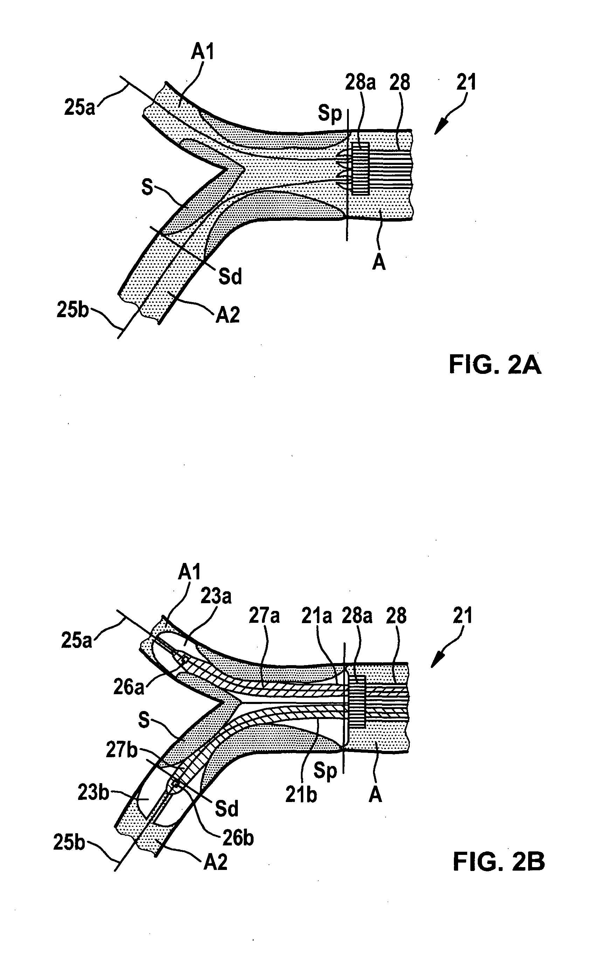

Fig. 2A bis 2D illustrieren die Funktionsweise einer Kathetervorrichtung 21 gemäß einer Ausführungsform der Erfindung in verschiedenen Betriebszuständen. In den Figuren ist lediglich das distale Ende der Kathetervorrichtung gezeigt. Deren weiterer Aufbau ergibt sich für den Fachmann unter Beachtung des Aufbaus des Hybridkatheters 1 aus Fig. 1, der für die Ausführung der Erfindung insbesondere im Wesentlichen "gedoppelt" wird. Vergleichbare Teile bzw. Abschnitte sind mit an Fig. 1 angelehnten Bezugsziffern bezeichnet.

Fig. 2A zeigt einen ersten Betriebszustand, in dem die Kathetervorrichtung 21 mit ihrem distalen Ende - markiert durch einen Röntgenmarker 28a als distalen Abschluss eines gemeinsamen Katheterschafts 28 - bis an das proximale Ende Sp einer Stenose S im Verzweigungsbereich einer Arterie A vorgeschoben ist. Aus dem distalen Ende der Kathetervorrichtung 21 sind bereits ein erster und zweiter Führungsdraht 25a, 25b in jeweils einen der Äste A1 bzw. A2 der Arterie A vorgeschoben. Die Führungsdrähte 25a und 25b haben unterschiedlichen Durchmesser, beispielsweise ca 0,356 mm (0,014") und 0,457 (0,018"). Die Bestimmung der geeigneten Position des distalen Endes des Außenschaftes der Kathetervorrichtung 21 für die nachfolgenden Schritte erfolgt durch Röntgenkontrolle anhand des Röntgenmarkers 28a.

Die Rollenmembranen werden nun über den (jeweiligen) Lueranschluss 3b mit Druck beaufschlagt. Dieser Druck bewirkt, dass sich die Rollmembran ausrollen möchte und sorgt so für eine Zugkraft auf den Innenschaft am distalen Ende, die proportional zum Rollmembrandruck ist. Gleichzeitig wird der Innenballon weiter zusammengepresst. Das Durchqueren der Stenose wird stark erleichtert,
- da es keine Relativbewegung zwischen der Rollmembran außen und der Stenose gibt,
- da die Rollmembran außen die Rollmembran innen nur auf einer Linie berühren kann, und somit die Reibkraft innerhalb der Rollmembran minimal bleibt,
- und da der Innenballonkatheter und Draht zusätzliche Pushability liefern.

Es muss beachtet werden, dass der Führungsdraht im inflatierten Zustand des Rollballons an der Innenrollmembran haftet und somit beim Durchqueren der Stenose vorgeschoben wird. Besteht die Gefahr eine Perforierung distal der Stenose durch den Führungsdraht, dann muss die Rollmembran kurz deflatiert werden, um den Führungsdraht zurück ziehen zu können.

Fig. 2B zeigt einen Betriebszustand nach dem Durchrollen beider Äste A1 und A2 der stenotischen Arterie A , in dem aus dem gemeinsamen Katheterschaft 28, der am proximalen Ende Sp der Stenose positioniert ist, die distalen Enden eines ersten Katheterzweigs 21 a und eines zweiten Katheterzweigs 21b längs des jeweiligen Führungsdrahtes 25a bzw. 25b in die Arterien-Äste A1 bzw. A2 ausgefahren sind. Beide Katheterzweige 21a, 21b haben am distalen Ende jeweils eine Rollmembran 23a bzw. 23b, und diese sind in dem Fig. 2B gezeigten Betriebszustand bereits inflatiert und ausgerollt. Die beiden Äste der Stenose können gleichzeitig oder nacheinander durchrollt werden. Das atraumatische Durchrollen des stenotischen Bereiches wird jeweils am distalen Ende Sd der Stenose S im entsprechenden Ast durch den Bediener gestoppt, indem er den Innenballonkatheter an seinem proximalen Ende durch eine Klemmvorrichtung arretiert. Damit ist die Länge der beiden Katheterzweige unabhängig voneinander einstellbar. Neben den Rollmembranen haben die Katheterzweige 21a, 21b jeweils einen innenliegenden Ballon (Innenballon) 27a bzw. 27b. Diese sind in dem hier gezeigten Zustand noch im nicht expandierten Ausgangszustand.

Die beiden Katheterzweige 21a, 21b unterscheiden sich hinsichtlich ihrer funktionswesentlichen Dimensionen (nachstehend auch als Funktionsabmessungen bezeichnet), wobei in Fig. 2B zu erkennen ist, dass der Durchmesser der Rollmembran 23b, die im weiteren Arterien-Ast A2 platziert ist, größer als derjenige der Rollmembran 23a am anderen Katheterzweig 21a ist, der in den engeren Arterien-Ast A1 vorgeschoben ist. Die verwechslungssichere Einführung des Katheterzweigs mit den "richtigen" Funktionsabmessungen im entsprechenden Arterien-Ast wird dadurch gewährleistet, dass auch die Führungsdrahtlumina der beiden Katheterzweige derart unterschiedlichen Durchmesser aufweisen, dass der Führungsdraht 25b mit dem größeren Durchmesser sich nur in den Katheterzweig 21b mit dem Führungsdrahtlumen größeren Durchmessers vorschieben lässt und somit dessen Position vorgibt. Hieraus ergibt sich dann auch der korrekte Weg des Führungsdrahtes 25a mit dem kleineren Durchmesser und somit auch des Katheterzweigs 21a mit den kleineren Funktionsabmessungen.

Zur korrekten Bestimmung der distalen Position der Katheterzweige weisen diese jeweils einen Röntgenmarker 26a bzw. 26b auf (der nur in Fig. 2B gezeigt ist). Sobald der entsprechende Katheterzweig 21a/b den stenotischen Bereich durchrollt hat, wird er dann, durch Arretieren am proximalen Ende des gemeinsamen Katheterschaftes 28, am weiteren Ausrollen gehindert. Die Rollmembran muss vor dem Inflatieren des Innenballons deflatiert werden.

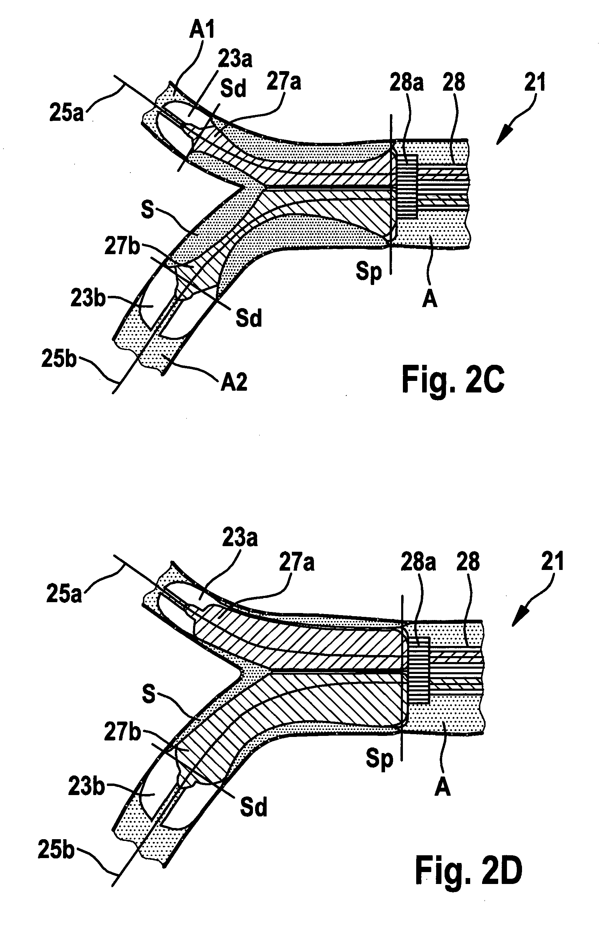

Wie Fig. 2C zeigt, wird in der derart vorbestimmten und gehaltenen Position der Katheterzweige der jeweilige stenotische Bereich durch Expansion der Ballone 27a, 27b mit deren Kraft geöffnet. Ein ungewolltes axiales Verlängern der Ballone 27a, 27b längs der jeweiligen Katheterzweig-Längsachse durch Verschieben des Innenschaftes kann z.B. durch eine Arretierung mittels Tuhoy Borst Dichtung der Katheterzweig-Schäfte am proximalen Katheterausgang verhindert werden.

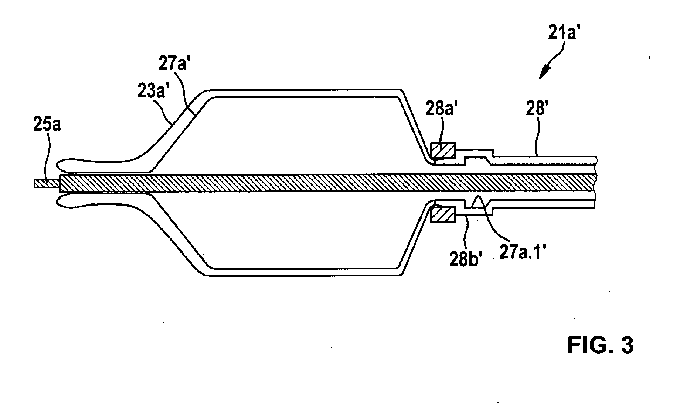

Zusätzlich kann sich der Innenballon, wie in Fig. 3 für einen der Katheterzweige als modifizierte Ausführung 21a' gezeigt, am distalen Ende des Katheterschachtes 28' durch einen Hinterschnitt 28b' im Außenschaft "verankern", da die Dilatationskräfte beim Maximaldruck in der Regel so groß werden, dass die proximale Arretierung nicht ausreicht. Der Innenschaft wird auf Zug und der Außenschaft auf Druck belastet, und diese reagieren entsprechend mit einer Verlängerung des Innenschaftes und Verkürzung des Außenschaftes, so dass die dilatierbare Ballonlänge ohne Gegenmaßnahme verlängert würde, was wiederum nicht erwünscht ist. Der sich im Hinterschnitt 28b' ausformende kleine Gegenballon 27a1' sorgt für ein axiales Gleichgewicht der Kräfte auf den Innenballon und stabilisiert damit seine Lage.

Fig. 2D zeigt einen aufgeweiteten Zustand der Stenose, also ein gewünschtes Therapieergebnis.

Zum Abschluss der Behandlung werden die Ballone 27a, 27b der beiden Katheterzweige 21a, 21b deflatiert, die angeschlossene Dilatationspumpe (nicht gezeigt) entsperrt und die Rollmembranen 23a, 23b unter leichtem Innendruck in den gemeinsamen Katheterschaft 28 zurückgerollt. Schließlich werden die Kathetervorrichtung 27 und die Führungsdrähte 25a, 25b aus dem therapierten Gefäß zurückgezogen.

## Patentansprüche

1. Kathetervorrichtung zur Aufweitung von Stenosen im Verzweigungsbereich von Körpergefäßen, umfassend zwei Katheterzweige (21a, 21b), **dadurch gekennzeichnet, dass**
die jeweils eine außenliegende distale Rollmembran (23a, 23b) aufweisen und am oder nahe dem distalen Ende des gemeinsamen Katheterschaftes (28) miteinander verbunden sind, in einem gemeinsamen Katheterschaft (28), und
wobei beide Katheterzweige (21a, 21b) jeweils einen innenliegenden Ballonkatheterteil aufweisen.

2. Kathetervorrichtung nach Anspruch 1, wobei die beiden Katheterzweige (21a, 21b) unterschiedliche Funktionsabmessungen, insbesondere einen unterschiedlichen Nutzdurchmesser der jeweiligen Rollmembran (23a, 23b) und/oder eines Innenballons (27a, 27b) des jeweiligen Ballonkatheterteils, aufweisen.

3. Kathetervorrichtung nach Anspruch 2, wobei die beiden Katheterzweige (21a, 21 b) Führungsdrahtlumen (25a, 25b) mit unterschiedlichem Durchmesser haben.

4. Kathetervorrichtung nach einem der vorangehenden Ansprüche, wobei die beiden Katheterzweige (21a, 21b) durch Verschweißen in einem Abschnitt ihres Umfangs miteinander verbunden sind.

5. Kathetervorrichtung nach einem der Ansprüche 1 - 4, wobei der Ballon (27a, 27b) jedes innenliegenden Ballonkatheterteils an oder nahe seinem distalen Ende einen Ballonende-Marker (26a, 26b) aufweist.

6. Kathetervorrichtung nach einem der Ansprüche 1 -5, wobei Mittel zur Lagefixierung des Innenballones (27b') jedes Ballonkatheterteils auf der Längsachse, insbesondere durch mindestens einen Hinterschnitt (28b') im Außenschaft, vorgesehen sind.

7. Kathetervorrichtung nach einem der Ansprüche 1 - 6, wobei die zwei Katheterzweige (21a, 21b) in ihrer Länge unabhängig voneinander einstellbar sind.

8. Kathetervorrichtung nach einem der Ansprüche 1 - 7, wobei die zwei Katheterzweige (21 a, 21 b) in ihrer Position unabhängig voneinander einstellbar sind.

9. Kathetervorrichtung nach einem der vorangehenden Ansprüche, wobei am Umfang der beiden Katheterzweige (21a, 21b), insbesondere auf deren Rollmembranabschnitten (23a, 23b), eine Wirkstoffbeschichtung und/oder eine Gleitmittelbeschichtung innen vorgesehen ist.

## Claims

1. A catheter device for expanding stenoses in the branch region of bodily vessels, comprising two catheter branches (21a, 21b), **characterised in that** the catheter branches each have an outer distal rolling membrane (23a, 23b) and are interconnected at or in the vicinity of the distal end of the common catheter shaft (28), in a common catheter shaft (28), and
wherein both catheter branches (21a, 21b) have an inner balloon catheter part.

2. The catheter device according to Claim 1, wherein the two catheter branches (21a, 21b) have different functional dimensions, in particular a different use diameter of the respective rolling membrane (23a, 23b) and/or of an inner balloon (27a, 27b) of the respective balloon catheter part.

3. The catheter device according to Claim 2, wherein the two catheter branches (21 a, 21b) have guide wire lumens (25a, 25b) having different diameters.

4. The catheter device according to one of the preceding claims, wherein the two catheter branches (21a, 21b) are interconnected by welding in a portion of their periphery.

5. The catheter device according to one of Claims 1 to 4, wherein the balloon (27a, 27b) of each inner balloon catheter part has a balloon end marker (26a, 26b) at or in the vicinity of its distal end.

6. The catheter device according to one of Claims 1 to 5, wherein means are provided for fixing the position of the inner balloon (27b') of each balloon catheter part on the longitudinal axis, in particular by at least one undercut (28b') in the outer shaft.

7. The catheter device according to one of Claims 1 to 6, wherein the lengths of the two catheter branches (21a, 21b) can be adjusted independently of one another.

8. The catheter device according to one of Claims 1 to 7, wherein the positions of the two catheter branches (21a, 21b) can be adjusted independently of one another.

9. The catheter device according to one of the preceding claims, wherein an active ingredient coating and/or a lubricant coating is provided internally on the periphery of the two catheter branches (21a, 21b), in particular on the rolling membrane portions (23a, 23b) thereof.

## Revendications

1. Dispositif de cathéter pour l'élargissement de sténoses dans la région de ramification de vaisseaux corporels, comprenant deux branches de cathéter (21a, 21b), **caractérisé en ce que**
elles comportent respectivement une membrane déroulante distale (23a, 23b) située à l'extérieur et sont reliées en une tige de cathéter commune (28), au niveau ou à proximité de l'extrémité distale de la tige de cathéter commune (28), et
dans lequel les deux branches de cathéter (21a, 21b) comportent respectivement une partie de cathéter à ballonnet du côté intérieur.

2. Dispositif de cathéter selon la revendication 1, dans lequel les deux branches de cathéter (21a, 21b) présentent des dimensions fonctionnelles différentes, en particulier un diamètre utile différent pour chacune des membranes déroulantes (23a, 23b) et/ou le ballonnet intérieur (27a, 27b) de chaque partie de cathéter à ballonnet.

3. Dispositif de cathéter selon la revendication 2, dans lequel les deux branches de cathéter (21a, 21b) possèdent des lumières de fil de guidage (25a, 25b) avec des diamètres différents.

4. Dispositif de cathéter selon l'une des revendications précédentes, dans lequel les deux branches de cathéter (21a, 21b) sont reliées entre elles par une soudure sur une partie de leur pourtour.

5. Dispositif de cathéter selon l'une des revendications 1 - 4, dans lequel le ballonnet (27a, 27b) de chaque partie de cathéter à ballonnet située du côté intérieur comporte un marqueur de fin de ballonnet (26a, 26b) au niveau ou à proximité de son extrémité distale.

6. Dispositif de cathéter selon l'une des revendications 1 - 5, dans lequel il est prévu des moyens pour fixer en position le ballonnet intérieur (27b') de chaque partie de cathéter à ballonnet sur l'axe longitudinal, en particulier par au moins une contre-dépouille (28b') dans la tige extérieure.

7. Dispositif de cathéter selon l'une des revendications 1 - 6, dans lequel les deux branches de cathéter (21a, 21b) peuvent être réglées indépendamment l'une de l'autre quant à leur longueur.

8. Dispositif de cathéter selon l'une des revendications 1 - 7, dans lequel les deux branches de cathéter (21a, 21b) peuvent être réglées indépendamment l'une de l'autre quant à leur position.

9. Dispositif de cathéter selon l'une des revendications précédentes, dans lequel il est prévu un revêtement de substance active et/ou un revêtement lubrifiant à l'intérieur, sur le pourtour des deux branches de cathéter (21a, 21b), en particulier sur leurs parties de membrane déroulante (23a, 23b).
